# EUROPEAN PATENT APPLICATION

(11) **EP 4 570 214 A1**
(43) Date of publication of application: **18.06.2025**
(21) Application number: 23216753.6
(22) Date of filing: 14.12.2023
(51) Int. Cl.: A61C 17/22, A46B 15/00, A61C 17/34, A61C 19/06, A61N 5/06

(54) **AN ORAL CARE DEVICE**

(71) Applicant: Koninklijke Philips N.V., 5656 AG Eindhoven (NL)
(72) Inventor: FRACKOWIAK, Bruno Jean François, Eindhoven (NL); VAN DEN DUNGEN, Wilhelmus Andreas Marinus Arnoldus, Eindhoven (NL); PAULUSSEN, Elvira Johanna Maria, Eindhoven (NL)
(74) Representative: Philips Intellectual Property & Standards

(57) **Abstract**

A mechanism for delivering light treatment to one or more dental regions. A proposed oral care device includes at least one light source, for generating light for performing a dental treatment, and an active light steering system. The active light steering system receives light generated by the light source(s) and steers the light out of the oral care device. The position(s) and/or direction(s) at which light is output from the oral care device are controllable using the active light steering system.

## Description

### FIELD OF THE INVENTION

The present disclosure relates to the field of oral care, and in particular to oral care devices able to provide lighting for application to dental areas.

### BACKGROUND OF THE INVENTION

There is an increasing interest and demand for oral care devices that are able to perform effective cleaning of one or more dental areas. Continued improvement to oral care devices is considered important for improving hygiene and reducing a risk of disease or infection in the mouth. Examples of oral care devices include toothbrushes, oral irrigators, RF cleaning units, UV cleaning units and/or dental mouthpieces.

One area of recent research and development relates to the application of light, particularly purple/violet and/or ultraviolet light, to dental areas. The application of light (i.e., "light treatment") has been shown to provide anti-inflammatory and antibacterial benefits for dental areas.

There is an ongoing desire to improve oral care devices with light application functionality.

### SUMMARY OF THE INVENTION

The invention is defined by the claims.

According to examples in accordance with an aspect of the invention, there is provided an oral care device for providing dental care to one or more dental regions of a subject.

The oral care device comprises: one or more light sources configured to generate light for application to the one or more dental areas of a subject; and an active light steering system configured to controllably adjust one or more positions from which and/or one or more directions in which light is output from the oral care device.

The present disclosure thereby provides an oral care device having a light steering system that facilitates (active) control over the direction(s) and/or position(s) in which light is output from the oral care device. This can, for instance, be utilized to control the direction of light according to some predetermined pattern, e.g.: to match a predefined brushing routine; to align with or be tailored for a specific user and/or oral environment; and/or to respond to feedback for the light treatment.

In particular, the active light steering system facilitate active/dynamic control over the size and/or location of one or more irradiated regions of the mouth of the subject during use of the oral care device. In other words, by facilitating the manipulation of the location(s)/position(s) and/or direction(s) of output light from the oral care device, the size and/or location of one or more dental areas of the subject (being areas that are treated by the oral care device) can be controlled.

It is noted that controlling the size of an irradiated region of the mouth, e.g., by controlling the number of positions and/or directions in which light is output from the oral care device, also facilitates control over the irradiance of the dental areas. This can be used to advantage to reduce a risk of overheating or burning any specific dental region.

For the avoidance of doubt, it is noted that the proposed oral care device may take the form of any known oral care device, and is not limited to any specific type or oral care device. Examples of oral care devices that may form embodiments of the proposed approach include toothbrushes, oral irrigators, RF cleaning units, UV cleaning units and/or dental mouthpieces.

The active light steering system may comprise a plurality of discrete light steering elements, each discrete light steering element being switchable between at least: an active mode, in which the discrete light steering element is configured to direct light out from the oral care device at a respective light output position of the oral care device; and an inactive mode, in which the discrete light steering element is configured to prevent or restrict the emission of light out from the oral care device at the respective light output position. The respective light output position of each discrete light steering element is different to the respective light output position of each other discrete light steering element.

Thus, there may be a finite or discrete number of output locations for light from the oral care device. The use of discrete light steering elements facilitates accurate control over the position at which light is output form the oral care device, e.g., as there is reduced or less reliance upon accurate and precise steering of the output light (as the positions or locations are known in advance).

In some examples, each discrete light steering element comprises an electrochromic material with a controllable scattering coefficient and/or reflectance. This provides a mechanism for well controlled adjustment and/or modification to the position(s) and/or direction(s) of output light.

In some examples, each discrete light steering element is a different layer of electrochromic material with a controllable scattering coefficient. This provides a relatively compact active light steering system, as the thickness of each discrete light steering element can be kept relatively small.

In some examples, each discrete light steering element is a different controllable mirror with adjustable reflectivity. This provides an alternative mechanism for controlling or adjusting the position(s) and/or direction(s) of output light.

In some examples, the active lighting steering system comprises one or more beam angle control elements, wherein each beam angle control element is configured to controllably adjust a beam angle of a respective first portion of light output from the oral care device. This provides a mechanism for adjusting or manipulating the irradiance of a region illuminated by the oral care device. By facilitating control over the beam angle, then adjustment or modifications can be made to reduce or attenuate localized heating of the mouth of the subject.

In some examples, the active light steering system comprises one or more beam direction control elements, each beam direction control element being configured to control a direction of a respective second portion of light output from the oral care device. Each beam direction control elements may comprise a respective mirror having an adjustable angle.

This technique provides a mechanism for finely or precisely tuning the direction of light output from the oral care device, e.g., for precise control over the irradiated dental region.

In some examples, the one or more light sources is configured to emit first light in a respective first direction; and the active light steering system is configured to control (or steer) a majority of the first light to be emitted out of the oral care device as second light, wherein the second light is output from the oral care device at one or more angles greater than 20° from first direction.

This approach aids to distance the light source(s) from tissue in the mouth, thereby reducing a risk of localized heating within the mouth.

In some examples, the active light steering system is configured to control or steer no less than 90% to form second light. In other words, the majority of the first light may comprise no less than 90% of the first light.

The oral care device may further comprise a control arrangement configured to: receive feedback data identifying a location of one or more desired dental regions with respect to the oral care device; and control the operation of active light steering system responsive to the received feedback data. This approach provides a mechanism for feedback control of the position(s) and/or direction(s) of output light. This can be used, for instance, to track locations that would benefit from light irradiation and/or to monitor for and respond to any undesired effects of the light irradiation (e.g., overheating). By responding to feedback information, the oral care device is able to adapt the position(s) and/or location(s) of output light to perform improved light treatment.

In some examples, the control arrangement is configured to control the operation of active light steering system to direct light towards the location of the one or more desired dental regions identified in the received feedback data. This advantageously performs a function of irradiating desired or target dental regions.

The oral care device may be formed as a mouthpiece. However, the oral care device is not restricted or limited to being a mouthpiece. For instance, the oral care device may take the form of a toothbrush, an oral irrigation device (e.g., a water flosser), an RF cleaning unit and so on.

In some examples, the oral care device may further comprise one or more brushing elements mounted on a mount, wherein the active light steering system is positioned on the mount, so as to move with a motion of the mount.

The oral care device may further comprise a mount moving system configured to move the mount to perform brushing with the one or more brushing elements.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
- Fig. 1: provides a schematic overview of a proposed oral care device;
- Fig. 2: illustrates an example active light steering system;
- Fig. 3: illustrates another example active light steering system;
- Fig. 4: illustrates another example active light steering system;
- Fig. 5: illustrates another example active light steering system;
- Fig. 6: illustrates a method for controlling an active light steering system; and
- Fig. 7: illustrates a proposed oral care device.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for delivering light treatment to one or more dental regions. A proposed oral care device includes at least one light source, for generating light for performing a dental treatment, and an active light steering system. The active light steering system receives light generated by the light source(s) and steers the light out of the oral care device. The position(s) and/or direction(s) at which light is output from the oral care device are controllable using the active light steering system.

The present disclosure thereby provides an oral care device for which the position and/or size of regions irradiated by light are controllable using an active light steering system. This increases a flexibility and adaptability of performing light treatment in the mouth.

The present invention describes a system for generating and steering light to specific areas in the mouth, in order to provide light treatment dental care in the mouth.

Embodiments provide an oral (health)care device containing one or more light sources and an active light steering system designed to controllably adjust the direction and/or angle of light emission out of the oral care device, e.g., to facilitate the targeting of light towards particular areas of the mouth where irradiance is needed/desired.

Example oral (health)care devices include mouthpieces, brushes, oral irrigation devices, water-based devices, moving light devices, RF cleaning units, UV cleaning units and so on. Any example of such oral (health)care devices may include the herein proposed one or more light sources and active light steering system.

Embodiments also disclose mechanisms or approaches for controlling the operation of the active light steering system, particularly providing data or information to be used to adapt parameters influencing the steering mechanism. More particularly, it is emphasized that the active light steering system is configured such that, during use of the oral care device, the direction and/or angle of light emission is able to be controlled, adjusted or modified throughout the use of the oral care device. Various advantages and techniques for such a concept are disclosed later in the description.

A variety of different optical elements can be used to define the active lighting steering system, including: variable optical scatterers based on electrical switchable optical properties; liquid crystal devices for control beam direction, beam angle or beam shape based on switchable refractive index; electrochromic devices for controlling optical properties as transmission, absorption and reflectance; electro-optical or acousto-optical modulators that can control beam deflection angle; a micro-electromechanical system (MEMS) with electronically driven optical beam steering micromirrors; metamaterials or metasurfaces and plasmon scatterers to scatter light; nanocrystals with switchable light output; (biocompatible scattering changing materials that can be used safely in the mouth; and/or (biodegradable) quantum dot or polymer dot crystalline materials. The following description will provide a number of examples for the use and implementation of such optical elements.

Proposed approaches provide an oral (health)care device in which light can be steered in real time towards specific target areas in the mouth (e.g. gumline and interdental cavity). In this way, it is possible to compensate for brushing motion and/or user adjustments of the device during an oral healthcare routine by way of the active or dynamic light steering. This facilitates the maintenance of always illuminated a desired target area.

Moreover, proposed approaches facilitate the steering of light to be patient specific. In particular, light steering may be responsive to a sensed target and/or inflamed areas (e.g., detected using a consumer device or a clinician-operated imaging system). In this way, light steering can be delivered to the patient based on the mapped target and/or inflamed areas within their mouth.

In the context of the present disclosure, a dental area is a part of the mouth of the subject, e.g., (a portion of) a tooth, (a portion of) a gum, (a portion of) a gumline or the like. More particularly, a dental area is a part or region of the mouth that would benefit from light treatment.

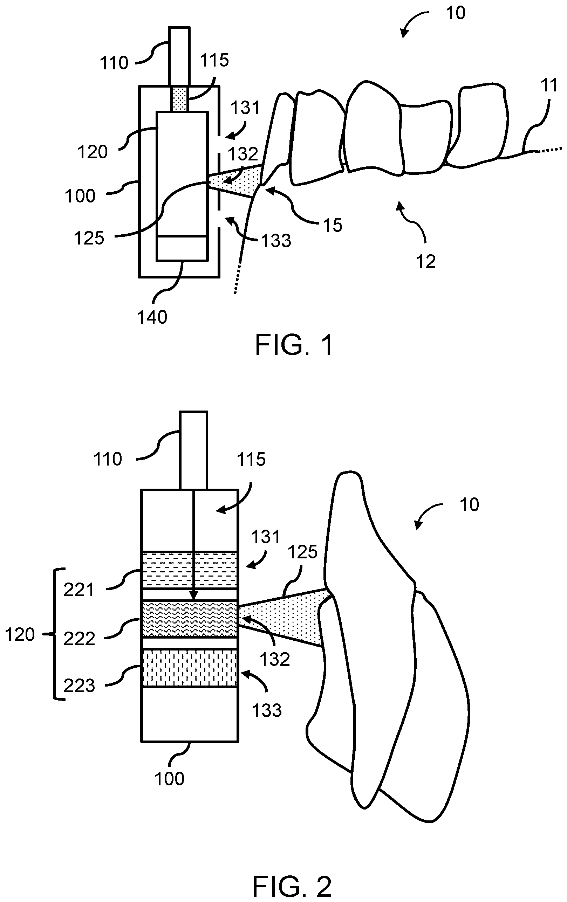

Fig. 1 conceptually illustrates a proposed oral care device 100 within an oral environment 10 (i.e., within the mouth of a subject). For the sake of contextual understanding, example gums 11 and teeth 12 of the oral environment are partially illustrated.

The oral care device 100 comprises at least one light source 110 (e.g., a light source 110) and an active light steering system 120.

The light source 110 is configured to generate (first) light 115 for application to one or more dental areas 15 of a subject. The (first) light 115 has oral care or oral health properties, e.g., a particular intensity and/or wavelength(s) for performing dental care when used to irradiate one or more dental regions of the subject. In particular, the light 115 may have anti-inflammatory, anti-bacterial and/or anti-microbial properties or functionality. For instance, the light 115 may comprise blue, violet and/or ultraviolet light. In some examples, each light source may comprise a semiconductor laser and/or LED for generating light for performing dental care.

The active light steering system 120 is configured to adjust or modify the position(s) and/or direction(s) from which light is output from the oral care device 100, e.g., towards the dental area(s) 15 of the subject. Thus, the active light steering system 120 receives the (first) light 115 emitted by the light source 110 and controls or defines the direction(s) and/or position(s) of output light 125 from the oral care device.

In this way, the active light steering system 120 is able to control the position(s) and/or direction(s) from which light is output from the oral care device towards the mouth of the subject. In this way, the active light steering system is able to respond to feedback or other instructions, e.g., indicating a change in the desired output position(s) and/or direction(s), to thereby adjust properties of light is output from the oral care device 100. Similarly, the active light steering system could be controlled or appropriated to tune or tailor the emission of output light to a specific individual or a specific situation of the individual (e.g., to target one or more inflamed or dirty dental regions).

In the illustrated example, the oral care device comprises a plurality of output locations 131, 132, 133. The active light steering system 120 may be configured to control out of which of these plurality of output locations 131, 132, 133, light is emitted from the oral care device (e.g., towards the dental areas).

This approach facilitates adjustment of the location of light emitted out of the oral care device. This functionality can be exploited, for example, to maintain an illuminance of a target dental area even if/when the oral care device changes position with respect to the mouth or oral environment.

The oral care device 100 may further comprise a control arrangement 140 configured to control the operation of the active light steering system. Although illustrated as separate elements, in practice, the control arrangement may form part of the active light steering system 120. In this way, the control arrangement 140 is able to control the direction(s) and/or position(s) of output light 125 from the oral care device.

The control arrangement 140 may be configured to receive one or more signals carrying information used by the control arrangement to control the position(s) and/or direction(s) of output light from the oral care device. For instance, the one or more signals may comprise feedback data (e.g., in the form of a feedback signal) and/or instructions (e.g., in the form of an instruction signal), which is/are used to control the position(s) and/or direction(s) from which light is output from the oral care device.

The control arrangement may be electrically communicatively coupled to the active light steering system in order to control the operation of the active light steering system.

A number of approaches for facilitating the control of the position(s) and/or direction(s) from which light is output from the oral care device are hereafter described. The skilled person will recognize that any combination of the proposed approaches can be used to advantage.

In one approach, the active light steering system comprises a plurality of discrete light steering elements. Each discrete light steering element is switchable between at least an active mode and an inactive mode. When operating in an active mode, the discrete light steering element is configured to direct light out from the oral care device at a respective light output position of the oral care device. When operating in an inactive mode, the discrete light steering element is configured to prevent or restrict the emission of light out from the oral care device at the respective light output position. The respective light output position of each discrete light steering element is different to the respective light output position of each other discrete light steering element. In this way, the active light steering system 120 is able to control out of which of a plurality of (discrete) positions light is emitted from the oral care device.

Each discrete light steering element may comprise an electrochromic material with a controllable scattering coefficient and/or reflectance.

Fig. 2 conceptually illustrates one approach for defining a plurality of discrete light steering elements.

In particular, Fig. 2 illustrates an oral care device 100 in which the active light steering system 120 comprises a plurality of light scattering layers 221, 222, 223 (each) having a controllable or adjustable scattering coefficient. Thus, each discrete light steering element is a different layer of electrochromic material with a controllable scattering coefficient.

For instance, each light scattering layer 221, 222, 223 may comprise or be formed from appropriately configured electroactive material, e.g., electrochromic material and/or liquid crystals. More particularly, such non-uniform scattering properties can be obtained by layering techniques within the oral care device, e.g., using biocompatible liquid crystals.

Each light scattering layer 221, 222, 223 is configured to be activatable, e.g., by an electrical signal controllably provided by a control arrangement (not illustrated in Fig. 2). When activated, the light scattering layer 221, 222, 223 will have an increased scattering coefficient compared to when inactivated. More particularly, when activated, the light scattering layer may scatter light and, when deactivated, the light scattering layer may scatter negligible or no light. The (de)activation of each light scattering layer may be controlled by the control arrangement (not visible in Fig. 2).

Each light scattering layer 221, 222, 223 is coupled to an exterior (surface) of the oral care device 100 at a respective light output position 131, 132, 133 such that, when a particular light scattering layer 222 is activated, light 125 is emitted out of the oral care device at the respective light output position by the increased scattering of the light scattering layer. Similarly, when the light scattering layer is deactivated, no or negligible light is output at the respective light output position. In the illustrated example, one light scattering layer 222 is activated, with two light scattering layers 221, 223 being inactivated.

Thus, to facilitate steering of the direction/position of light, the oral care device 100 comprises multiple layers with variable scattering particles. By activating one specific layer (e.g., with an electrical signal), the scattering coefficient of the layer can be increased locally, thus steering the light towards the output position for that layer, and thereby to the specific dental area in front of or facing said output position.

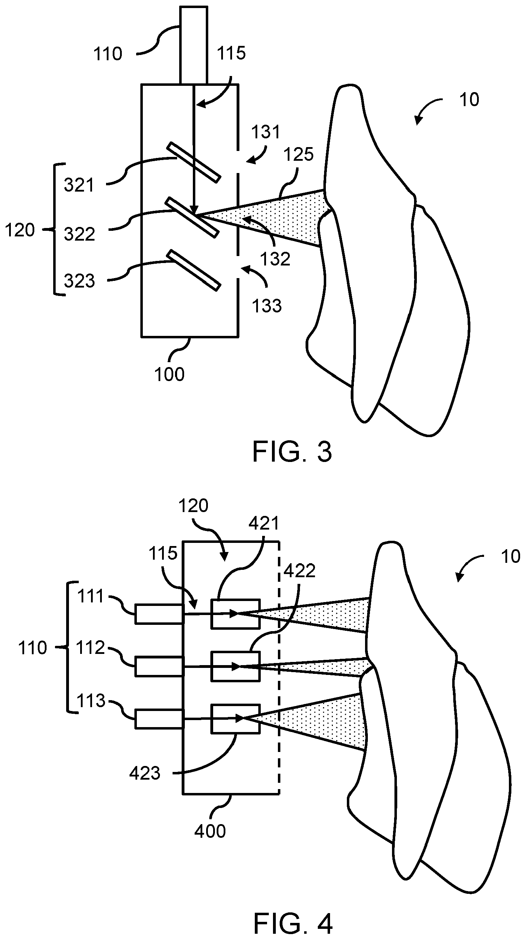

Fig. 3 conceptually illustrates another approach for defining a plurality of discrete light steering elements.

In this approach, the active light scattering system 120 of the oral care device 100 comprises a plurality of controllable mirrors 321, 322, 323, which may also be known as switchable mirrors. Each controllable mirror has an adjustable reflectivity. Thus, each discrete light steering element is a different controllable mirror with adjustable reflectivity.

For instance, each controllable mirror 321, 322, 323 may comprise or be formed from electroactive material, e.g., electrochromic material and/or liquid crystals. More particularly, such materials facilitate control over the reflective properties of each controllable mirror, e.g., to controllably switch each controllably mirror between being (substantially) transmissive and (substantially) reflective.

Each controllable mirror 321, 322, 323 is configured to be activatable, e.g., by an electrical signal controllably provided by a control arrangement (not illustrated in Fig. 3). When activated, the controllable mirror 321, 322, 323 will have an increased reflection. More particularly, when activated, the controllable mirror 321, 322, 323 may reflect light and, when deactivated, the controllable mirror 321, 322, 323 may reflect negligible or no light.

Each controllable mirror 321, 322, 323 is positioned to receive light 115 emitted by the light source 110 (if not previously intercepted by a previous controllable mirror that has been controlled to be reflective). When activated, each controllable mirror reflects the light 115 such that it is redirected to output the oral care device at a respective (different) light output position 131, 132, 133. Similarly, when deactivated, each controllable mirror reflects no or negligible light at the respective light output position 131, 132, 133 no or negligible light is output at the respective light output position. In the illustrated example, one controllable mirror 322 is activated, with two controllable mirrors 321 323 being inactivated.

Thus, layers of (e.g., electrochromic) switchable mirrors can be embedded in the oral care device. Light can therefore be steered by adapting reflectance (e.g., in real time). This prevents or restricts the light being transmitted across some surfaces, thus avoiding or reducing the illumination of non-target dental areas.

Examples of suitable materials for forming a controllable mirror (i.e., having an adjustable reflectivity - also known as switchable mirrors) are well established in the art, such as those disclosed by: Richardson, Thomas J. "New electrochromic mirror systems." Solid State Ionics 165.1-4 (2003): 305-308; Kamimori, Tadatoshi, Junichi Nagai, and Mamoru Mizuhashi. "Electrochromic devices for transmissive and reflective light control." Solar energy materials 16.1-3 (1987): 27-38; and/or Baucke, F. G. K., K. Bange, and T. Gambke. "Reflecting electrochromic devices." Displays 9.4 (1988): 179-187.

In the embodiments illustrated in Figures 2 and 3, light is emitted by the light source 110 in a direction substantially perpendicular to the (average) direction in which light is output from the oral care device.

More particularly, the one or more light sources is configured to emit first light ("input light") in a respective first direction. The active light steering system is configured to steer the first light to produce second light or "output light" that is emitted out of the oral care device. In preferred examples, the active light steering system is configured to control the second light to be emitted out of the oral care device at one or more angles greater than 20° from the first direction, e.g., at one or more angles greater than 40° from the first direction, e.g., substantially perpendicular to the first direction.

Preferably, a majority of the first light (e.g., >90% of the first light) is redirected by the active light steering system to form the second light. Thus, in some examples, a minority (e.g., <10%) of the first light is absorbed, scattered, internally reflected or otherwise forms stray light, i.e., without being redirected and output as the second light.

By positioning the light source(s) to emit light in this orientation, the light source(s) can be distanced from the teeth, gums and lips. This reduces a risk of localized heating at one or more dental areas, reducing a risk of damage to the subject.

However, this positional relationship is not essential to all embodiments. Rather, the one or more light sources may be positioned to be substantially parallel to the (average) direction of light output from the oral care device.

Fig. 4 illustrates another example of an oral care device 400.

In this example, the active lighting steering system 120 comprises beam angle control elements 421, 422 423. Each beam angle control element is configured to controllably adjust a beam angle of a respective first portion of light output from the oral care device. The operation of each beam angle control element may be controlled by a control arrangement (not illustrated in Fig. 4), e.g., using appropriate electrical signals.

Thus, each beam angle control element 421, 422, 423 receives light from a (respective) light source 111, 112, 113. Each beam angle control element adjusts or modifies the beam angle of light output from the beam angle control element. This facilitates the control over the beam angle of light (and therefore directions of light) from each of one or more output locations of the oral care device.

Examples of beam angle control elements will be readily apparent to the skilled person. For instance, each beam angle control element may comprise a liquid crystal or similar element. As another example, each beam angle control element may comprise a pair of (micro)lenses whose separation distance can be controlled, e.g., using a MEMS, piston, worm drive or other similar system.

The beam angle influences or affects the total and peak irradiance of the tissue (i.e., the size of the dental area illuminated by a portion of the light generated by a light source), e.g., peak irradiance can be modelled as being proportional to the smallest beam angle. Moreover, larger emitting angles results in a shadowing effect occurring in interdental spaces, thus reducing the irradiance level.

Localized heating at irradiated dental areas can be reduced (e.g., hot spots can be avoided) by increasing the beam angle, at the expense of less irradiance and therefore less effective light treatment. Thus, by controlling the beam angle of light output via each beam angle control element, it is possible to balance between irradiance and heating of a dental area.

Fig. 4 illustrates a preferred example in which the beam angle of light incident upon the gums is relatively large, e.g., compared to the beam angle of light incident upon the teeth or gumline, to reduce or mitigate a risk of burning the gums (which are more sensitive than the teeth). The beam angle of light incident upon the gumline is relatively small, indicating that the gumline is region at which irradiation is most desired (e.g., to perform effective cleaning of the oral environment).

Approaches for distinguishing or discriminating between different parts of the oral environment are later described, and can be used to particular advantage in this embodiment.

Although Fig. 4 illustrates an oral care device 400 comprising a plurality of beam angle control elements 421, 422, 423, it will be appreciated that one or more of these can be omitted (e.g., the oral care device 400 may instead comprise a single beam angle control element).

In the embodiment illustrated in Fig. 4, light is emitted by the light source 110 in a direction or beam substantially parallel to the (average) direction in which light is output from the oral care device. However, in other embodiments, light is emitted by the light source 110 in a plane substantially perpendicular to the (average) direction in which light is output from the oral care device. This could, for instance, be achieved through the use of a mirror or other light guiding system (such as fiber optics). The use of such other light guiding systems may increase the material cost and manufacturing complexity (due to a need for good alignment for effective light coupling), and may therefore be less preferable. However, such approaches may reduce a risk of localized heating to tissue of the subject.

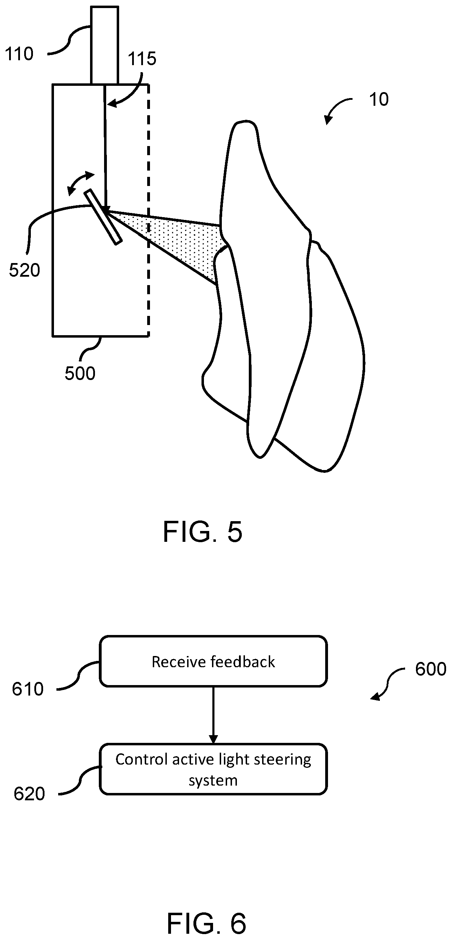

Fig. 5 illustrates another example of an oral care device 500.

In this example, the active light steering system comprises a beam direction control element 520. The beam direction control element is configured to control a direction of a respective second portion of light output from the oral care device. Thus, the beam direction control element receives light emitted by the one or more light sources 110 and controls the direction at which the received light is redirected out of the oral care device 500.

In the illustrated example, the beam direction control element comprises a mirror having an adjustable angle. By way of example, the beam direction control element may be embodied as a MEMs device. Such a MEMs device can be readily integrated in an oral care device 500 due to their relatively small size. Other suitable approaches for controlling the angle of a mirror within an oral care device will be apparent to the appropriately skilled person, e.g., a through use of a (air/liquid) bladder or piston to manipulate the angle of the mirror.

Although Fig. 5 illustrates an oral care device 500 comprising a single beam direction control element, it will be appreciated that the oral care device 500 may, in practice, comprise a plurality of beam direction control elements, e.g., each embodied as a respective mirror having an adjustable angle.

In some further examples, if the oral care device 500 comprises a plurality of beam direction control elements, each beam direction control element (e.g., respective mirror) may also function as a discrete light steering element as previously described. For instance, each beam direction control element may be switchable between an active mode and an inactive mode, as previously described. As an example, each beam direction control element may be formed as a respective switchable/controllable mirror having an adjustable angle. Thus, each beam direction control element may be a different controllable mirror with adjustable reflectivity and adjustable angle.

The foregoing description provides a number of techniques and approaches for controlling the position(s) and/or direction(s) in which light is output by an oral care device. This facilitates control over the size, intensity and/or location of one or more dental areas that are irradiated with light by the oral care device during use of the oral care device.

The skilled person will appreciate that the above-described approaches provide a non-exhaustive list of options and examples for one or more elements of an active light steering system, and that a wide variety of other options will be understood by the skilled person.

It has previously been described how the oral care device may comprise a control arrangement that controls the operation of the active light steering system. In particular, the control arrangement may comprise a processor that receives one or more input signals and outputs one or more output signals to the active light steering system so as to control the operation thereof.

The control arrangement may therefore employ or comprise a processor to carry out its functionality. The processor can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Proposed embodiments are particularly advantageous when combined with a control arrangement that adjusts or modifies the position(s) and/or direction(s) in which light is output by an oral care device during an oral care session (e.g., a treatment or brushing session) of the oral care device. In particular, proposed embodiments are particularly advantageous when the control arrangement is configured to adjust the operation of the active light steering system throughout an operation session of the oral care device, where an operation session is an oral care session such as an oral cleaning session or an oral treatment session.

The control arrangement may, for instance, adjust the position(s) or direction(s) of output light according to a predetermined pattern or routine, responsive to a user indication or user and/or responsive to feedback (e.g., automatically sensed information about the oral care session).

By way of example, the control arrangement may adjust the position(s) or direction(s) of output light such that (during use of the oral care device) the output light moves in a predetermined, random or pseudorandom pattern over the dental region or oral environment of the subject. One use for such a technique is to reduce a risk of localized heating at any specific location of the subject. Such a technique can also be used to account for tolerance in the positioning of the oral care device placement and/or deformation, e.g., to move the position(s) and/or direction(s) of output light such that the irradiated portion of the oral environment includes the dental region(s) and an error margin around the dental region(s).

As another example, the control arrangement may adjust the position(s) or direction(s) of output light responsive to a user input, e.g., such that the subject or other user can control the size, intensity and/or location of one or more dental areas that are irradiated with light by the oral care device during its use. This provides a high degree of flexibility of use. As an example, the user may indicate (via a user input) that a temperature of a currently irradiated region is too high or uncomfortable. The control arrangement may respond to this user input by adjusting the position(s) or direction(s) of output light, e.g., to change the dental region(s) irradiated by the oral care device and/or the size of the irradiated dental regions (to reduce the irradiance of dental regions).

As another example, the control arrangement may adjust the position(s) or direction(s) of output light such that (during use of the oral care device) the output light moves synchronously with a brushing motion of the oral device. For instance, the oral care device may comprise one or more brushing elements mounted on a mount, wherein the active light steering system is positioned on the mount, so as to move with a motion of the mount. The oral care device may further comprise a mount moving system configured to move the mount to perform brushing with the one or more brushing elements. The control arrangement may control the output light to move synchronously with the movement of the mount by the mount moving system (which can be predefined or dynamic). This can help maintain the irradiance of a desired dental region using the output light.

In some examples, the control arrangement is configured to adjust the position(s) and/or direction of output light responsive to feedback. The feedback may be automatically generated by the oral care device, i.e., be independent of any direct user input during operation.

Thus, although not illustrated, the oral care device may comprise one or more sensors or sensor units configured to generate feedback data responsive to one or more properties of the oral environment and/or oral care device (e.g., a movement of the oral care device, a temperature and/or an amount of reflected light).

Turning back to Fig. 1, it has previously been described how the oral care device 100 may comprise a control arrangement 140. Fig. 6 illustrates a method 600 that may be performed by such a control arrangement.

The control arrangement 140 may be configured to receive (in a step 610) feedback data identifying a location of one or more desired dental regions with respect to the oral care device; and control (in a step 620) the operation of active light steering system responsive to the received feedback data.

Thus, the control arrangement may effectively perform dynamic steering of the position(s) and/or direction(s) in which light is output from the oral care device responsive to information that identifies or otherwise indicates the position of a desired dental region (which can be alternatively labelled a target dental region).

In this way, the control arrangement may be configured to control the operation of active light steering system to direct light towards the location of the one or more desired dental regions identified in the received feedback data.

A desired dental region may be one for which light treatment is desired, such as a gumline (e.g., where brushing is less effective or bristles are less likely to reach effectively).

One suitable example of feedback data is reflected light data, which is data generated by one or more optical sensors responsive to light (initially output from the oral care device) which is reflected from the oral environment. Thus, to provide feedback on the amount of light reaching the desired dental region(s), the oral care device may comprise one or more optical sensors configured to generate reflected light data responsive to light reflected from the oral environment.

Accordingly, the oral care device may comprise one or more optical sensors configured to monitor and/or sense reflected light to generate reflected light data. Examples of suitable optical sensors are well known to the skilled person such as those comprising one or more photodetectors (e.g., MSM photodetectors, CCDs, phototransistors photodiodes, photoresistors and so on), or image sensors (e.g., CCD image sensors or active-pixel sensors). The control arrangement may control the operation of active light steering system responsive to the reflected light data.

It is recognized that light (e.g., violet light) will be absorbed to a greater extent by gums than by teeth. In other words, teeth are more reflective than gums. The control arrangement can use this information to discriminate or predict whether or not the gums or the teeth are being irradiated. This information can be used to target particular dental regions, e.g., regions of the gum, or the gumline. More particularly, the feedback data can be used in a feedback loop to dynamically steer the light output from the oral care device to become incident and/or focused upon the desired region(s), e.g., as indicated by a user or preset. As an example, it may be more preferably to irradiate the gums than the teeth or it may be preferable to target irradiation of the gumline for improved dental hygiene.

It is also recognized that higher levels of blood in the gums will increase its absorbance, thus reducing the reflected light intensity. High levels of blood in gums are indicative of inflammation (i.e., gingivitis). It is therefore possible to discriminate between portions of the gums that are inflamed (i.e., have higher levels of blood) and noninflamed portions. It would be advantageous to perform treatment of inflamed regions of the gum. Accordingly, reflected light data can be used to target inflamed regions of the gum(s), e.g., by changing the position(s) and/or direction(s) of output until it is incident upon the dark(est) area(s) of the gums, e.g., using a feedback loop.

These provide example techniques for embodying a control arrangement configured to control the operation of active light steering system to direct light towards the location of the one or more desired dental regions identified in the received feedback data.

Another suitable example of feedback data is temperature data, e.g., identifying a temperature of one or more dental regions irradiated by the oral care device during use. This information can be used to determine whether the irradiated dental region(s) are becoming too hot, e.g., there is localized overheating.

Accordingly, the oral care device may comprise one or more temperature sensors configured to monitor and/or sense a temperature to produce temperature data. Examples of suitable temperature sensors (also known as thermometers) are well known to the skilled person, e.g., thermistors, thermocouples, RTDs and so on. The control arrangement may control the operation of active light steering system responsive to the temperature data.

The control arrangement may be, for instance, configured to monitor the temperature data to identify if a threshold is breached. Responsive to a threshold being breached, the control arrangement may adjust or modify the position(s) and/or direction(s) in which light is output from the oral care device, i.e., to reduce a risk of perform the same/similar irradiation of the dental region(s) and potentially damaging the subject's mouth. The threshold may be predefined or defined by a user. Thus, the temperature data may be monitored or used as feedback to maintain a temperature of the dental region(s) within a desired bound or boundary.

As another example, the temperature data may be monitored to aid in discriminating between different regions of the mouth (e.g., between teeth and gums), as teeth are likely to be cooler than gums (due at least to lack of blood flow and their material composition). This provides another example technique for embodying a control arrangement configured to control the operation of active light steering system to direct light towards the location of the one or more desired dental regions identified in the received feedback data.

Other suitable uses for temperature data for feedback control will be apparent to the skilled person. For instance, the temperature data may be monitored for any sudden spikes or changes, which may indicate a risk of damage.

Another suitable example of feedback data is movement data, e.g., identifying a movement of the oral care device during use. This information can be used, for instance, to identify a change in positional relationship between the oral care device and the oral environment (e.g., due to user movement, brushing using the oral care device and so on). The movement information may, for instance, be used to maintain an irradiance on one or more target dental regions or to track dental regions that are being irradiated.

Accordingly, the oral care device may comprise one or more movement sensors configured to monitor and/or sense a movement of the oral care device to produce movement data. Suitable examples of movement sensors are well known in the art, including accelerometers, gyroscopes and/or inclinometers.

The control arrangement may be, for instance, configured to monitor the movement data to track changes in the position between the oral care device and the oral environment. This movement data may, for instance, be used to maintain an irradiation on a same target dental region, through appropriate changes to the direction(s) and/or position(s) of output light. Thus, the control arrangement is appropriately configured to control the operation of active light steering system to direct light towards the location of the one or more desired dental regions identified in the received feedback data. More particularly, the feedback data can be used to identify a relative position of a previously irradiated dental region (e.g., a desired dental region) after a movement of the oral care device.

Any one or more of the above described examples of feedback data may be used in embodiments. Thus, feedback data may comprise reflected light data, temperature data and/or movement data. Of course, these are a non-exhaustive set of examples of forms of feedback data, and other examples will be apparent to the skilled person.

Although previously described examples control the direction(s) and/or position(s) of output light during use of the oral care device (e.g., during the performance of an oral care, cleaning or treatment session), this is not essential. For instance, the control arrangement may be used to predefine the direction(s) and/or position(s) of output light prior to use of the oral care device, i.e., prior to the light source(s) being activated. This can be used to direct the output light towards clinician or user-identified dental regions of interest.

By way of example, the positional relationship between the oral care device and the mouth of the subject may be known. This may, for instance, be as result of a registration of the oral care device to the subject and/or the predetermined shape of the oral care device, e.g., defining where the oral care device can be positioned.

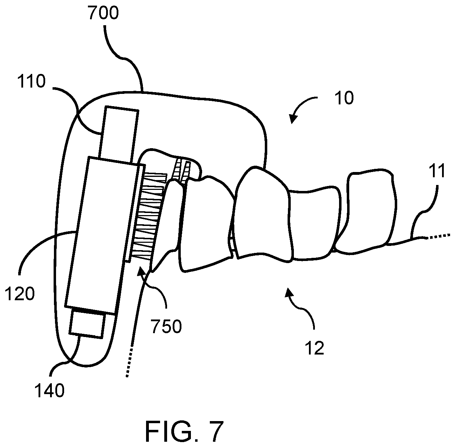

Fig. 7 illustrates a cross-section of an oral care device 700, which takes the form of a mouthpiece.

A mouthpiece typically has an arcuate (e.g., U-shaped or J-shaped) structure with upper and lower tooth-receiving channels, and typically include curved rows of bristles 750 which follow the shape of the tooth-receiving channels. These allow rapid and thorough cleaning of teeth with reduced effort for the user.

By integrating the light source(s) and active light steering system in such a mouthpiece, improved performance of teeth cleaning and/or treatment can be achieved. In particular, as the movement or manipulation of mouthpiece during a cleaning operation is significantly less than a toothbrush (e.g., negligible), then it is acceptable for the mouthpiece to have an increased size.

Thus, in accordance with one or more embodiments, the oral care device may comprise a mouthpiece for being received in the oral cavity of a user.

The mouthpiece may be U-shaped and may include upper and lower tooth-receiving channels, with a biting surface disposed between the two channels, forming a base for each of the channels. It may alternatively be a J-shaped partial mouthpiece in other examples.

The mouthpiece may comprise a plurality of cleaning elements for rubbing against tooth surfaces during an operation session. The cleaning elements may comprise cleaning filaments. The cleaning elements may be bristles or bristle bundles. The oral care device may include a movement generator for driving oscillatory movement of the cleaning elements (e.g., bristles) over the tooth surfaces.

However, the oral care device is not restricted or limited to being a mouthpiece. For instance, the oral care device may take the form of a toothbrush, an oral irrigation device (e.g., a water flosser), an RF cleaning unit and so on.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. An oral care device (100, 400, 500, 700) for providing dental care to one or more dental regions of a subject, the oral care device comprising:
one or more light sources (110) configured to generate light for application to the one or more dental areas of a subject; and
an active light steering system (120, 520) configured to controllably adjust one or more positions from which and/or one or more directions in which light is output from the oral care device.

2. The oral care device of claim 1, wherein the active light steering system comprises a plurality of discrete light steering elements, each discrete light steering element being switchable between at least:
an active mode, in which the discrete light steering element is configured to direct light out from the oral care device at a respective light output position of the oral care device; and
an inactive mode, in which the discrete light steering element is configured to prevent or restrict the emission of light out from the oral care device at the respective light output position,
wherein the respective light output position of each discrete light steering element is different to the respective light output position of each other discrete light steering element.

3. The oral care device of claim 2, wherein each discrete light steering element comprises an electrochromic material with a controllable scattering coefficient and/or reflectance.

4. The oral care device of claim 3, wherein each discrete light steering element is a different layer of electrochromic material with a controllable scattering coefficient.

5. The oral care device of claim 3, wherein each discrete light steering element is a different controllable mirror with adjustable reflectivity.

6. The oral care device of any of claims 1 to 5, wherein the active lighting steering system comprises one or more beam angle control elements, wherein each beam angle control element is configured to controllably adjust a beam angle of a respective first portion of light output from the oral care device.

7. The oral care device of any of claims 1 to 6, wherein the active light steering system comprises one or more beam direction control elements, each beam direction control element being configured to control a direction of a respective second portion of light output from the oral care device.

8. The oral care device of claim 7, wherein each beam direction control element comprises a respective mirror having an adjustable angle.

9. The oral care device of any of claims 1 to 8, wherein:
the one or more light sources is configured to emit first light in a respective first direction; and
the active light steering system is configured to control a majority of the first light to be emitted out of the oral care device as second light, wherein the second light is output from the oral care device at one or more angles greater than 20° from first direction.

10. The oral care device of claim 9, wherein the active light steering system is configured to control no less than 90% to form second light.

11. The oral care device of any of claims 1 to 10, further comprising a control arrangement configured to:
receive feedback data identifying a location of one or more desired dental regions with respect to the oral care device; and
control the operation of active light steering system responsive to the received feedback data.

12. The oral care device of claim 11, wherein the control arrangement is configured to control the operation of active light steering system to direct light towards the location of the one or more desired dental regions identified in the received feedback data.

13. The oral care device of any of claims 1 to 12, wherein the oral care device is formed as a mouthpiece

14. The oral care device of any of claims 1 to 13, further comprising one or more brushing elements mounted on a mount,
wherein the active light steering system is positioned on the mount, so as to move with a motion of the mount.

15. The oral care device of claim 14, further comprising a mount moving system configured to move the mount to perform brushing with the one or more brushing elements.
